(19) Europäisches Patentamt
European Patent Office
Office européen des brevets

(11) Veröffentlichungsnummer: **0 315 869**
**A2**

(12) # EUROPÄISCHE PATENTANMELDUNG

(21) Anmeldenummer: **88118111.9**

(22) Anmeldetag: **31.10.88**

(51) Int. Cl.[4]: **C07D 207/34 , C07D 405/04 , A01N 43/36**

(30) Priorität: **09.11.87 DE 3737983**

(43) Veröffentlichungstag der Anmeldung:
**17.05.89 Patentblatt 89/20**

(84) Benannte Vertragsstaaten:
**BE CH DE FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Wollweber, Detlef, Dr.**
**Paul-Ehrlich-Strasse 17**
**D-5600 Wuppertal 1(DE)**
Erfinder: **Brandes, Wilhelm, Dr.**
**Eichendorffstrasse 3**
**D-5653 Leichlingen(DE)**

(54) **3-Cyano-4-phenyl-pyrrol-Derivate.**

(57) 3-Cyano-4-phenyl-pyrrol-Derivate der Formel (I)

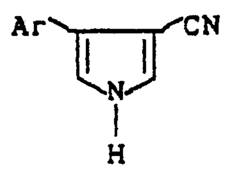

(I)

in welcher
Ar für drei- bis fünffach substituiertes Phenyl steht,
sowie ihre Verwendung in Schädlingsbekämpfungsmitteln und neue Zwischenprodukte.

Die neuen 3-Cyano-4-phenyl-pyrrol-Derivate können z.B. hergestellt werden, indem man geeignete substituierte Zimtsäurenitrile mit geeigneten Sulfonylmethylisocyaniden in Gegenwart einer Base umsetzt. Die zu verwendenden substituierten Zimtsäurenitrile sind auch neu und können z.B. hergestellt werden, indem man geeignete substituierte Aniline mit Acrylnitril umsetzt und anschließend dehydrohalogeniert oder geeignete Benzaldehyde mit Cyanessigsäure kondensiert und gleichzeitig decarboxyliert.

EP 0 315 869 A2

## 3-Cyano-4-phenyl-pyrrol-Derivate

Die Erfindung betrifft neue 3-Cyano-4-phenyl-pyrrol-Derivate, ein Verfahren zu ihrer Herstellung und ihre Verwendung in Schädlingsbekämpfungsmitteln und neue Zwischenprodukte.

Es ist bekannt, daß bestimmte 3-Cyano-4-phenyl-pyrrole, wie beispielsweise die Verbindung 3-Cyano-4-(2,3-dichlorphenyl)-pyrrol, fungizide Wirksamkeit besitzen (vgl. z.B. EP 236 272).

Die Wirksamkeit dieser vorbekannten Verbindungen ist jedoch insbesondere die niedrigen Aufwandmengen und Konzentrationen nicht in allen Anwendungsgebieten völlig zufriedenstellend.

Es wurden neue 3-Cyano-4-phenyl-pyrrol-Derivate der allgemeinen Formel (I),

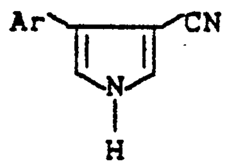

(I)

in welcher
Ar für drei- bis fünffach substituiertes Phenyl steht,
gefunden.

Weiterhin wurde gefunden, daß die neuen 3-Cyano-4-phenyl-pyrrol-Derivate der allgemeinen Formel (I),

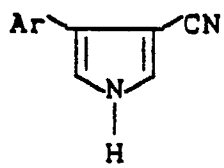

(I)

in welcher
Ar für drei- bis fünffach substituiertes Phenyl steht,
erhält, wenn man substituierte Zimtsäurenitrile der Formel (II),
Ar-CH = CH-CN (II)
in welcher
Ar die oben angegebene Bedeutung hat,
mit Sulfonylmethylisocyaniden der Formel (III),
$R-SO_2-CH_2-NC$ (III)
in welcher
R für Alkyl oder für gegebenenfalls substituiertes Aryl steht,
in Gegenwart einer Base und gegebenenfalls in Gegenwart eines Verdünnungsmittels umsetzt.

Schließlich wurde gefunden, daß die neuen 3-Cyano-4-phenyl-pyrrol-Derivate der allgemeinen Formel (I) eine gute Wirkung gegen Schädlinge besitzen.

Überraschenderweise zeigen die erfindungsgemäßen 3-Cyano-4-phenyl-pyrrol-Derivate der allgemeinen Formel (I) eine erheblich bessere fungizide Wirksamkeit als z.B. die aus dem Stand der Technik bekannten 3-Cyano-4-phenyl-pyrrole, wie beispielsweise die Verbindung 3-Cyano-4-(2,3-dichlorphenyl)-pyrrol, welches chemisch und wirkungsmäßig naheliegende Verbindungen sind.

Die erfindungsgemäßen 3-Cyano-4-phenyl-pyrrol-Derivate sind durch die Formel (I) allgemein definiert. Bevorzugt sind Verbindungen der Formel (I), bei welchen
Ar für drei- bis fünffach, gleich oder verschieden substituiertes Phenyl steht, wobei als Substituenten genannt seien: Halogen, insbesondere Fluor, Chlor oder Brom, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen, jeweils zweifach verknüpftes Dioxyalkylen oder Halogendioxyalkylen mit jeweils 1 oder 2 Kohlenstoffatomen und gegebenenfalls 1 bis 4 gleichen oder verschiedenen Halogenatomen.

Besonders bevorzugt sind Verbindungen der Formel (I), bei welchen
Ar für drei- bis fünffach, gleich oder verschieden substituiertes Phenyl steht, wobei als Substituenten

besonders bevorzugt sind: Fluor, Chlor, Brom, Methyl, Methoxy, Methylthio, Trifluormethyl, Trifluormethoxy, Trifluormethylthio, jeweils zweifach verknüpftes Dioxymethylen oder Difluordioxymethylen.

Ganz besonders bevorzugt sind Verbindungen der Formel (I), bei welchen
Ar für drei- bis fünffach, gleich oder verschieden substituiertes Phenyl steht, wobei mindestens ein Substituent Fluor ist und wobei als weitere Substituenten infrage kommen: Chlor, Brom, Methyl, Methoxy, Methylthio, Trifluormethyl, Trifluormethoxy, Trifluormethylthio oder Difluordioxymethylen.

Im einzelnen seien außer den bei den Herstellungsbeispielen genannten Verbindungen die folgenden 3-Cyano-4-phenyl-pyrrol-Derivate der allgemeinen Formel (I) genannt:

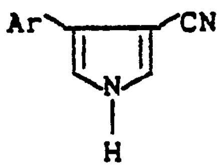

(I)

| Ar | Ar | Ar |
|---|---|---|
| 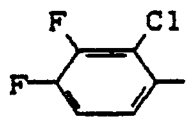 | 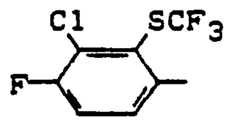 | 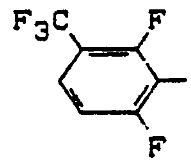 |
| 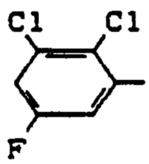 | 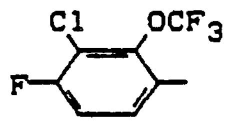 | 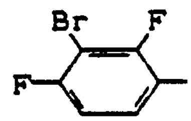 |
| 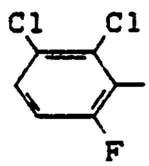 | 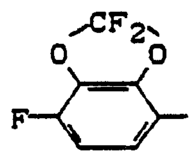 | 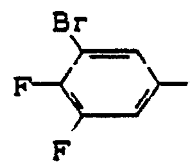 |
| 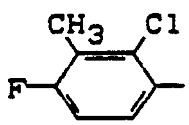 | 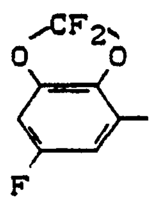 | 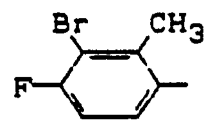 |
| 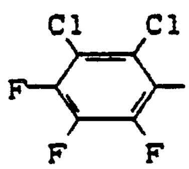 | 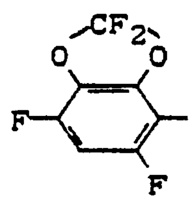 | 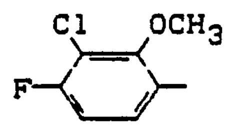 |
| 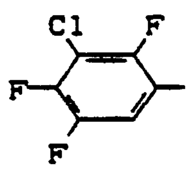 | 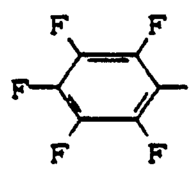 | 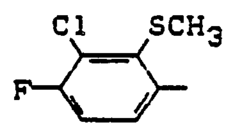 |

| Ar | Ar | Ar |
|---|---|---|
| 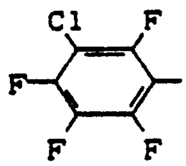 | 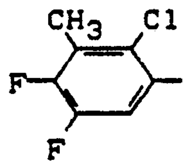 | 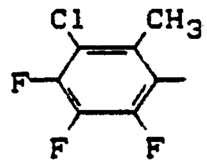 |
| 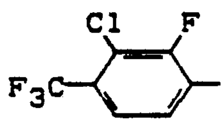 | 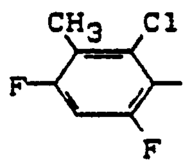 | 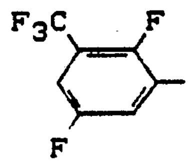 |
| 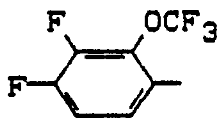 | 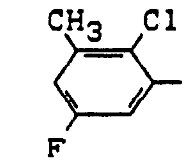 | 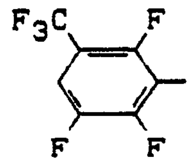 |
| 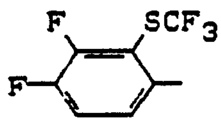 | 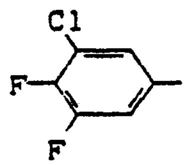 | 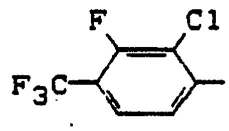 |

Verwendet man beispielsweise 3,4-Dichlor-2-fluorzimtsäurenitril und p-Toluolsulfonylmethylisocyanid als Ausgangstoffe und Natriumhydrid als Base, so läßt sich der Reaktionsablauf des erfindungsgemäßen Verfahrens durch das folgende Formelschema darstellen:

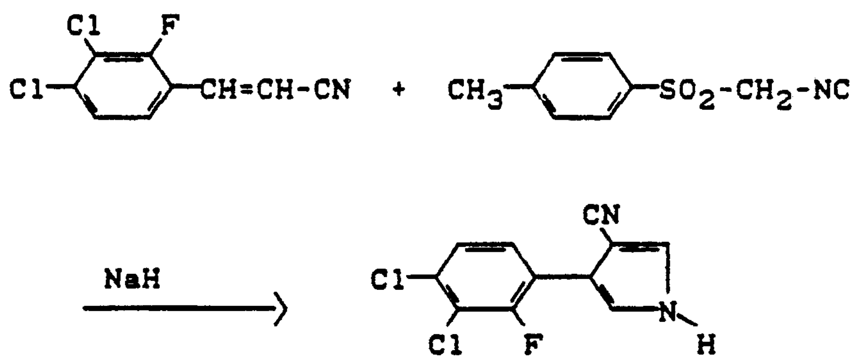


Die zur Durchführung des erfindungsgemäßen Verfahrens als Ausgangsstoffe benötigten substituierten Zimtsäurenitrile sind durch die Formel (II) allgemein definiert. In dieser Formel (II) steht Ar vorzugsweise für diejenigen Reste, die bereits im Zusammenhang mit der Beschreibung der erfindungsgemäßen Stoffe der Formel (I) für diesen Substituenten genannt wurden.

Die substituierten Zimtsäurenitrile der Formel (II) sind neu. Man erhält sie jedoch in Analogie zu bekannten Verfahren (vgl. z.B. DE-OS 2 927 480), beispielsweise wenn man

a) substituierte Aniline der Formel (IV),

$Ar\text{-}NH_2$ (IV)

in welcher

Ar die oben angegebene Bedeutung hat,
zunächst in einer ersten Stufe mit Acrylnitril unter üblichen Diazotierungsbedingungen, beispielsweise in Gegenwart von Natriumnitrit und Salzsäure, und in Gegenwart eines geeigneten Metallsalz-Katalysators, wie beispielsweise Kupfer-II-chlorid oder Kupfer-II-oxid, und gegebenenfalls in Gegenwart eines geeigneten Verdünnungsmittels, wie beispielsweise Aceton oder wasser, bei Temperaturen zwischen - 20 °C und 50 °C umsetzt ("Meerwein-Arylierung"; vgl. hierzu auch Organic Reactions 11, 189 [1960]; Organic Reactions 24, 225 [1976] oder C. Ferri "Reaktionen der organischen Synthese" S. 319, Thieme Verlag Stuttgart 1978) und dann in einer 2. Stufe die so erhältlichen substituierten α-Chlor-β-phenyl-propionitrile der Formel (V),

$$\begin{array}{l} \qquad\quad Cl \\ \qquad\quad | \\ Ar\text{-}CH_2\text{-}\ CH\ \text{-}CN \qquad (V) \end{array}$$

in welcher
Ar die oben angegebene Bedeutung hat,
mit Basen, wie beispielsweise Triethylamin oder Diazabicycloundecen, in üblicher Art und Weise gegebenenfalls in Gegenwart eines Verdünnungsmittels, wie beispielsweise Tetrahydrofuran, bei Temperaturen zwischen 0 °C und 50 °C dehydrohalogeniert (vgl. auch die Herstellungsbeispiele) oder alternativ,

b) substituierte Benzaldehyde der Formel (VI),

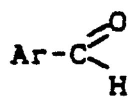

(VI)

in welcher
Ar die oben angegebene Bedeutung hat,
mit Cyanessigsäure der Formel (VII),
$NC\text{-}CH_2\text{-}COOH$ (VII)
in üblicher Art und Weise in Gegenwart einer Base, wie beispielsweise Piperidin oder Pyridin, und gegebenenfalls in Gegenwart eines geeigneten Verdünnungsmittels, wie beispielsweise Pyridin, bei Temperaturen zwischen 50 °C und 120 °C kondensiert und gleichzeitig decarboxyliert (vgl. z.B. "Organikum" S. 571/572; 15. Auflage; VEB Deutscher Verlag der Wissenschaften Berlin 1981 sowie die Herstellungsbeispiele).

Substituierte Aniline der Formel (IV) sind teilweise bekannt (vgl. z.B. J. org. Chem. 39, 1758-1761 [1974]; J. med. Chem. 12, 195 - 196 [1969] oder US-PS 3 900 519).

Neu sind z.B. die substituierten Aniline der Formel (IVa)

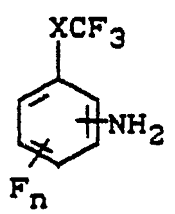

(IVa)

in der
X für Sauerstoff oder Schwefel und
n für 2 stehen.

Sie sind Gegenstand einer eigenen, nicht vorveröffentlichten Anmeldung (vgl. deutsche Patentanmeldung P 3 737 985 vom 09. Nov. 1987).

Ein allgemein anwendbares Verfahren zur Herstellung von Verbindungen der Formel (IVa)

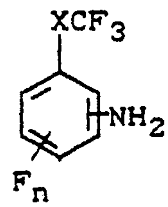

(IVa)

in der
X für Sauerstoff oder Schwefel und
n für 2 stehen,
ist dadurch gekennzeichnet, daß man Verbindungen der Formel (VIII)

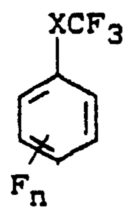

(VIII)

in der
X und n die bei Formel (IVa) angegebene Bedeutung haben,
nitriert und die so erhaltenen Nitroverbindungen reduziert.

Die in dieses Verfahren einzusetzenden Fluor enthaltenden Trifluormethoxy- und Trifluormethylthiobenzole sind bekannt.

Die Nitrierung kann mit üblichen Nitriermitteln durchgeführt werden, beispielsweise mit Gemischen aus Salpeter- und Schwefelsäure. Die Temperatur kann dabei beispielsweise im Bereich von 0 bis 80 °C liegen, vorzugsweise liegt sie zwischen 20 und 50 °C. Das Nitriermittel kann man beispielsweise in Mengen einsetzen, bei denen im Reaktionsgemisch 0,8 bis 1,5 Mole Nitrieragenz pro Mol Ausgangsverbindung gebildet werden. Vorzugsweise läßt man sich 1 bis 1,1 Mol Nitrieragenz pro Mol Ausgangsverbindung bilden. Die Nitrierung kann gegebenenfalls in Gegenwart eines inerten organischen Lösungsmittels durchgeführt werden. Geeignet ist beispielsweise Methylenchlorid.

Die daran anschließende Reduktion kann chemisch, d.h. beispielsweise mit reduzierend wirkenden Metallen oder Metallsalzen durchgeführt werden. Geeignet sind beispielsweise Eisen, Zink, Zinn, Zinn(II)-chlorid und Titan(III)chlorid. Derartige Reduktionsmittel werden vorzugsweise in der stöchiometrisch erforderlichen Menge eingesetzt. Für eine derartige Reduktion können die Nitroverbindungen z.B. so eingesetzt werden, wie sie bei der Nitrierung anfallen oder danach isoliert werden. Die Reduktion kann man auch katalytisch mit Wasserstoff durchführen, wobei z.B. Katalysatoren eingesetzt werden können, die Metalle enthalten oder daraus bestehen. Geeignet sind beispielsweise die Metalle der VIII. Nebengruppen des Periodischen Systems der Elemente, insbesondere Palladium, Platin und Nickel. Die Metalle können in elementarer Form oder in Form von Verbindungen vorliegen, sowie in besonders aktivierten Formen, z.B. in Form von Raney-Metallen oder aufgebracht als Metall oder Metallverbindung auf Trägermaterialien. Bevorzugt ist Raney-Nickel und Palladium auf Kohle und Alumiumoxid.

Vorzugsweise wird die katalytische Reduktion in Gegenwart eines Lösungsmittels durchgeführt. Geeignet sind beispielsweise Alkohole und Ether, wie Methanol, Ethanol und Tetrahydrofuran. Die katalytische Reduktion kann beispielsweise bei Temperaturen im Bereich 0 bis 80 °C und beispielsweise bei Wasserstoffdrucken im Bereich 1 bis 100 bar durchgeführt werden. Überschüsse an Wasserstoff sind im allgemeinen nicht kritisch.

Für die katalytische Reduktion werden vorzugsweise säurefreie Nitroverbindungen eingesetzt. Gegebenenfalls sind letztere also von Säuren zu befreien, z.B. durch Waschen mit Wasser oder Neutralisation mit einer Base.

Die Aufarbeitung des nach der chemischen Reduktion oder der katalytischen Hydrierung vorliegenden Reaktionsgemisches kann beispielsweise so erfolgen, daß man zunächst gegebenenfalls vorhandene feste Bestandteile abfiltriert und das Filtrat, gegebenenfalls nach einer Wäsche mit Wasser, destilliert. Falls als Reaktionsprodukt ein Gemisch von Isomeren anfällt, kann man dieses durch Feindestillation trennen.

Verbindungen der Formel (IVa) mit Fluor in o- und p-Stellung zur Aminogruppe kann man auch herstellen, indem man Verbindungen der Formel (IX)

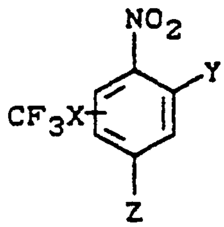

(IX)

in der
X für Sauerstoff oder Schwefel und
Y und Z für Chlor stehen,
mit Kaliumfluorid in Gegenwart von Tetramethylensulfon umsetzt, wobei vorhandenes Chlor durch Fluor ersetzt wird, und anschließend eine Reduktion durchführt, wobei die Nitrogruppe in eine Aminogruppe überführt wird.

Verbindungen der Formel (IX) sind bekannt (vgl. Houben-Weyl, Methoden der organischen Chemie E 4, S. 633ff).

Pro Äquivalent in der Verbindung der Formel (IX) gegen Fluor auszutauschendes Chlor kann man beispielsweise 0,5 bis 3 Mol Kaliumfluorid einsetzen. Vorzugsweise beträgt diese Menge von 1.2 bis 1.5 Mol. Tetramethylensulfon fungiert als Lösungsmittel und wird vorzugsweise mindestens in einer solchen Menge eingesetzt, daß ein gut rührbares Reaktionsgemisch vorliegt. Größere Mengen an Lösungsmittels stören nicht.

Geeignete Temperaturen für die Umsetzung mit Kaliumfluorid in Tetramethylensulfon sind beispielsweise solche im Bereich von 160 bis 230 °C. Bevorzugte Temperaturen sind solche von 180 bis 210 °C. Vorzugsweise führt man die Umsetzung in möglichst wasserfreiem Milieu durch. Dies kann man beispielsweise dadurch erreichen, daß man als letzte Komponente die Verbindung der Formel (IX) in sorgfältig getrockneter Form einsetzt und aus den vorher zusammengegebenen sonstigen Komponenten eine kleine Menge Tetramethylensulfon zusammen mit gegebenenfalls vorhandenem Wasser abdestilliert.

Nach Beendigung der Reaktion können im Reaktionsgemisch vorliegende Feststoffe und gegebenenfalls ganz oder teilweise das Tetramethylensulfon entfernt werden.

Die anschließende Reduktion der Nitro- zur Aminogruppe und die Aufarbeitung des dann vorliegenden Reaktionsgemisches kann so erfolgen, wie zuvor beim allgemein anwendbaren Verfahren zur Herstellung der Verbindungen der Formel (IVa) beschrieben worden ist.

Verbindungen der Formel (IVa) mit X = Sauerstoff können auch hergestellt werden indem man Verbindungen der Formel (X)

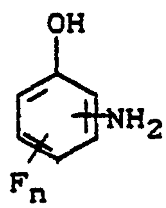

(X)

in der
n für 2 steht,
mit Tetrachlorkohlenstoff in Gegenwart von Fluorwasserstoff umsetzt, wobei die OH-Gruppe in eine $CF_3O$-Gruppe überführt wird.

Verbindungen der Formel (X) sind bekannt (vgl. FR-Pat. 2.446.805).

Pro Mol der jeweiligen Verbindung der Formel (X) kann man beispielsweise 1 bis 10 Mol Tetrachlorkohlenstoff und 5 bis 30 Mol Fluorwasserstoff einsetzen. Auch größere Überschüsse von Tetrachlorkohlenstoff und Fluorwasserstoff stören im allgemeinen nicht. Geeignete Reaktionstemperaturen sind beispielsweise solche im Bereich von 100 bis 150 °C. Dieses Verfahren führt man vorzugsweise unter Druck durch, beispielsweise indem man das entstehende Chlorwasserstoffgas erst oberhalb eines bestimmten Druckes entspannt. Dieser Druck kann beispielsweise bei 18 bis 60 bar liegen. Gegebenenfalls kann man zusätzlich ein inertes Gas aufdrücken, z.B. 1 bis 20 bar Stickstoff. Es ist vorteilhaft, während der Reaktion gut zu rühren.

Die Aufarbeitung des Reaktionsgemisches kann man beispielsweise so durchführen, daß man das

Reaktionsgemisch auf Raumtemperatur abkühlt, entspannt, überschüssigen Fluorwasserstoff und überschüssigen Tetrachlorkohlenstoff beispielsweise bei Temperaturen bis 80 °C abdestilliert, den Rückstand auf Eiswasser gibt, mit Natronlauge alkalisch stellt, die organische Phase mit Dichlormethan extrahiert und nach dem Trocknen einer Feindestillation unterwirft.

Weiterhin sind Fluor und/oder Chlor enthaltende Trifluormethylaminobenzole neu, bei denen sich die Trifluormethylgruppe in 1-Stellung, die Aminogruppe

a) in 2-Stellung

und zwei Fluoratome in 5- und 6-Stellung oder ein Fluoratom in 5-Stellung und ein Chloratom in 6-Stellung oder

b) in 3-Stellung

und zwei Fluoratome in 2- und 6-Stellung oder ein Chloratom in 2-Stellung und ein Fluoratom in 6-Stellung oder

c) in 4-Stellung und ein Fluoratom in 2-Stellung

und zusätzlich ein weiteres Fluoratom in 3- oder 6-Stellung oder zwei weitere Fluoratome in 5- und 6-Stellung oder ein weiteres Fluoratom in 6-Stellung und zwei Chloratome in 3- und 5-Stellung oder ein Chloratom in 3-Stellung oder zwei Chloratome in 3- und 5-Stellung oder

d) in 4-Stellung

und zwei Fluoratome in 3- und 5-Stellung oder zwei Chloratome in 2- und 3-Stellung oder ein Fluoratom in 3-Stellung und ein Chloratom in 5-Stellung,

befinden.

Auch diese Amine sind Gegenstand einer eigenen nicht vorveröffentlichten Anmeldung (vgl. deutsche Patentanmeldung P 3 737 986 vom 09.11.1987).

Bei diesen Verbindungen handelt es sich im einzelnen um 2-Amino-5,6-difluor-benzotrifluorid, 2-Amino-5-fluor-6-chlor-benzotrifluorid, 2,6-Difluor-3-amino-benzotrifluorid, 2-Chlor-3-amino-6-fluor-benzotrifluorid, 2,3-Difluor-4-amino-benzotrifluorid, 2,6-Difluor-4-amino-benzotrifluorid, 2,5,6-Trifluor-4-amino-benzotrifluorid, 2,6-Difluor-3,5-dichlor-4-amino-benzotrifluorid, 2-Fluor-3-chlor-4-amino-benzotrifluorid, 2-Fluor-3,5-dichlor-4-amino-benzotrifluorid, 3,5-Difluor-4-amino-benzotrifluorid, 2,3-Dichlor-4-amino-benzotrifluorid und 3-Fluor-4-amino-5-chlor-benzotrifluorid.

Ein bevorzugtes Verfahren zur Herstellung Fluor und/oder Chlor enthaltender Trifluormethylaminobenzole, bei denen sich die Trifluormethylgruppe in 1-Stellung, die Aminogruppe

a) in 2-Stellung

und zwei Fluoratome in 5- und 6-Stellung oder ein Fluoratom in 5-Stellung und ein Chloratom in 6-Stellung oder

b) in 3-Stellung

und zwei Fluoratome in 2- und 6-Stellung oder ein Chloratom in 2-Stellung und ein Fluoratom in 6-Stellung oder

c) in 4-Stellung und ein Fluoratom in 2-Stellung

und zusätzlich ein weiteres Fluoratom in 3- oder 6-Stellung oder zwei weitere Fluoratome in 5- und 6-Stellung oder ein weiteres Fluoratom in 6-Stellung und zwei Chloratome in 3- und 5-Stellung oder ein Chloratom in 3-Stellung oder zwei Chloratome in 3-und 5-Stellung oder

d) in 4-Stellung

und zwei Fluoratome in 3- und 5-Stellung oder zwei Chloratome in 2- und 3-Stellung oder ein Fluoratom in 3-Stellung und ein Chloratom in 5-Stellung be finden, ist dadurch gekennzeichnet, daß man entsprechende Fluor und/oder Chlor enthaltende Trifluormethylbenzole nitriert und die so erhaltenen Fluor und/oder Chlor enthaltenden Trifluormethylnitrobenzole reduziert.

Die in dieses Verfahren einzusetzenden Fluor und/oder Chlor enthaltenden, aber von Aminogruppen freien Trifluormethylbenzole, sind bekannt (vgl. z.B. J. Chem. Soc. C, (8), 1547-9 (1971)).

Die Nitrierung kann mit üblichen Nitriermitteln durchgeführt werden, beispielsweise mit Gemischen aus Salpeter-und Schwefelsäure. Die Temperatur kann dabei beispielsweise im Bereich von 0 bis 80 °C liegen, vorzugsweise liegt sie bei 20 bis 50 °C. Das Nitriermittel kann man beispielsweise in Mengen einsetzen, bei denen im Reaktionsgemisch 0,8 bis 1,5 Mole Nitrieragenz pro Mol Ausgangsverbindung gebildet werden. Vorzugsweise setzt man soviel Nitriermittel ein, daß sich 1 bis 1,1 Mol Nitrieragenz pro Mol Ausgangsverbindung bilden. Die Nitrierung kann gegebenenfalls in Gegenwart eines inerten organischen Lösungsmittels durchgeführt werden. Geeignet ist beispielsweise Methylenchlorid.

Die daran anschließende Reduktion kann chemisch, d.h. beispielsweise mit reduzierend wirkenden Metallen oder Metallsalzen durchgeführt werden. Geeignet sind beispielsweise Eisen, Zink, Zinn, Zinn(II)-chlorid und Titan(III)-chlorid. Derartige Reduktionsmittel werden vorzugsweise in der stöchiometrisch erforderlichen Menge eingesetzt. Für eine derartige Reduktion können die Nitroverbindungen z.B. so eingesetzt werden, wie sie bei der Nitrierung anfallen oder in gereinigter Form.

Die Reduktion kann man auch katalytisch mit Wasserstoff durchführen, wobei z.B. Katalysatoren eingesetzt werden können, die Metalle enthalten oder daraus bestehen. Geeignet sind beispielsweise die Metalle VIII. Nebengruppe des Periodensystems der Elemente, insbesondere Palladium, Platin und Nickel. Die Metalle können in elementarer Form oder in Form von Verbindungen vorliegen, sowie in besonders aktivierten Formen, z.B. in Form von Raney-Metallen oder als Metall oder Metallverbindung aufgebracht auf Trägermaterialien. Bevorzugt ist Raney-Nickel und Palladium auf Kohle und Aluminiumoxid.

Vorzugsweise wird die katalytische Reduktion in Gegenwart eines Lösungsmittels durchgeführt. Geeignet sind beispielsweise Alkohole und Ether, wie Methanol, Ethanol und Tetrahydrofuran. Die katalytische Reduktion kann beispielsweise bei Temperaturen im Bereich 10 bis 60 °C und beispielsweise bei Wasserstoffdrucken im Bereich 1 bis 100 bar durchgeführt werden. Überschüsse an Wasserstoff sind im allgemeinen nicht kritisch.

Für die katalytische Reduktion werden vorzugsweise säurefreie Nitroverbindungen eingesetzt. Nach der Herstellung sind sie also gegebenenfalls von Säuren zu befreien, z.B. durch Waschen mit Wasser oder Neutralisation mit einer Base.

Die Aufarbeitung des nach der chemischen Reduktion oder der katalytischen Hydrierung vorliegenden Reaktionsgemisches kann beispielsweise so erfolgen, daß man zunächst gegebenenfalls vorhandene feste Bestandteile ab filtriert und das Filtrat, gegebenenfalls nach einer Wäsche mit Wasser, destilliert. Falls als Reaktionsprodukt ein Gemisch aus Isomeren anfällt kann man diese gegebenenfalls durch Feindestillation trennen.

In einer Variante der zuvor beschriebenen katalytischen Reduktion wird in Gegenwart einer Base gearbeitet, beispielsweise in Gegenwart von Hydroxiden oder Carbonaten der Alkalimetalle oder tertiären Aminen. Bevorzugt sind tertiäre Amine wie Triethylamin oder Pyridin. Mit jeweils einem Äquivalent Base pro Mol der in die katalytische Reduktion eingesetzten Nitroverbindung kann aus letzterer während der katalytischen Reduktion zusätzlich ein Äquivalent Chloratome abgespalten werden. Man kann so beispielsweise aus einem Fluor und Chlor enthaltenden Nitrobenzotrifluorid ein von Chlor freies, Fluor enthaltendes Aminobenzotrifluorid erhalten.

Ein weiteres Verfahren speziell zur Herstellung von Fluor und/oder Chlor enthaltenden Trifluormethylaminobenzolen, bei denen sich die Trifluormethylgruppe in 1-Stellung, die Aminogruppe

a′) in 2-Stellung

und zwei Fluoratome in 5- und 6-Stellung oder ein Fluoratom in 5-Stellung und ein Chloratom in 6-Stellung oder

b′) in 4-Stellung und

ein Fluoratom in 2-Stellung

und zusätzlich ein weiteres Fluoratom in 3- oder 6-Stellung oder zwei weitere Fluoratome in 5- und 6-Stellung oder ein weiteres Fluoratom in 6-Stellung und zwei Chloratome in 3- und 5-Stellung oder ein Chloratom in 3-Stellung oder zwei Chloratome in 3-und 5-Stellung oder

c′) in 4-Stellung und zwei Fluoratome in 3- und 5-Stellung oder zwei Chloratome in 2- und 3-Stellung oder ein Fluoratom in 3-Stellung und ein Chloratom in 5-Stellung

befinden, ist dadurch gekennzeichnet, daß man entsprechende Fluor und/oder Chlor enthaltende, 2- und/oder 4-Halogen-trifluormethylbenzole bei erhöhtem Druck mit Ammoniak in Gegenwart eines organischen Lösungsmittels umsetzt.

Die in dieses Verfahren einzusetzenden 2- und/oder 4-Halogen-trifluormethylbenzole sind bekannt (vgl. EP 34.402).

Das Ammoniak kann in flüssiger oder gasförmiger Form zugesetzt werden, z.B. in Substanz (gasförmig oder flüssig) oder als wäßrige Lösung. Pro Mol in 2- und/oder 4-Stellung durch $NH_2$-Gruppen zu ersetzende Halogenatome kann man beispielsweise 1 bis 10 Mol Ammoniak verwenden. Bevorzugt beträgt diese Menge 3 bis 8 Mol. Geeignete Temperaturen für diese Umsetzung sind beispielsweise solche im Bereich von 80 bis 160 °C, bevorzugt sind solche im Bereich von 100 bis 130 °C. Die Umsetzung kann under dem sich im geschlossenen Gefäß bei Reaktionstemperatur einstellenden Eigendruck des Ammoniaks durchgeführt werden, der beispielsweise im Bereich von 10 bis 20 bar liegen kann. Man kann auch höhere Drucke anwenden, beispielsweise solche bis zu 100 bar.

Als Lösungsmittel für diese Umsetzung sind inerte oder weitgehend inerte organische Lösungsmittel der verschiedensten Art einsetzbar. Beispielsweise kommen in Frage: Alkohole, Ether, Sulfone und aromatische Kohlenwasserstoffe.

Aus dem nach der Umsetzung vorliegenden Reaktionsgemisch kann man das oder die gewünschten Reaktionsprodukte z.B. erhalten, indem man zunächst abkühlt und den Druck entspannt, dann das

Lösungsmittel entfernt und anschließend eine Destillation, vorzugsweise unter vermindertem Druck, durchführt.

Die weiterhin als Vorprodukte zur Herstellung der neuen Ausgangsprodukte der Formel (II) nach Variante b) benötigten Fluorbenzaldehyde der Formel (VI) sind größtenteils bekannt (vgl. z.B. Jap. Pat. 58-222 045 = C.A. 100: 209 288k) und ebenso ist die Cyanessigsäure der Formel (VII) allgemein bekannt (vgl. Organikum "Organisch-chemisches Grundpraktikum", Berlin 1977, VEB Deutscher Verlag der Wissenschaften, Seite 573).

Die zur Durchführung des erfindungsgemäßen Verfahrens als Ausgangsstoffe benötigten Sulfonylmethylisocyanide sind durch die Formel (III) allgemein definiert. In dieser Formel (III) steht $R^1$ vorzugsweise für Methyl oder für gegebenenfalls einfach substituiertes Phenyl, wie beispielsweise 4-Methylphenyl, 4-Chlorphenyl oder Phenyl.

Die Sulfonylmethylisocyanide der Formel (III) sind bekannt (vgl. z.B., Synthesis 1985, 400-402; Org. Syntheses 57, 102-106 [1977]; J. org. Chem 42, 1153-1159 [1977]; Tetrahedron Lett. 1972, 2367-2368).

Als Verdünnungsmittel zur Durchführung des erfindungsgemäßen Verfahrens kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Amide, wie Dimethylformamid, Dimethylacetamid, N-Methylformanilid, N-Methylpyrrolidon oder Hexamethylphosphorsäuretriamid, oder Sulfoxide, wie Dimethylsulfoxid.

Das erfindungsgemäße Verfahren kann gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl- $C_{13}/C_{15}$-alkylammoniumchlorid, Dibenzyl-dimethylammoniummethylsulfat, Dimethyl-$C_{12}/C_{14}$-alkyl-benzylammoniumchlorid, Tetrabutylammoniumhydroxid, 15-Krone-5, 18-Krone-6, Triethylbenzylammoniumchlorid, Trimethylbenzylammoniumchlorid.

Das erfindungsgemäße Verfahren wird vorzugsweise in Gegenwart einer geeigneten Base durchgeführt. Als solche kommen alle üblicherweise verwendbaren anorganischen und organischen Basen in Frage. Vorzugsweise verwendet man Alkalimetallhydride, -hydroxide, -amide, -alkoholate, -carbonate oder -hydrogencarbonate, wie beispielsweise Natriumhydrid, Natriumamid, Natriumhydroxid, Natriummethylat, Natriumethylat, Kalium-t-butylat, Natriumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens in einem größeren Bereich variiert werden. Im allgemeinen arbeitet man bei Temperaturen zwischen - 30 °C und + 120 °C, vorzugsweise bei Temperaturen zwischen - 20 °C und + 50 °C.

Zur Durchführung des erfindungsgemäßen Verfahrens setzt man pro Mol an substituierten Zimtsäurenitril der Formel (II) im allgemeinen 1.0 bis 2.0 Mol, vorzugsweise 1.0 bis 1.5 Mol an Sulfonylmethylisocyanid der Formel (III) und 1.0 bis 2.0 Mol, vorzugsweise 1.0 bis 1.5 Mol an Base ein.
Dabei ist es gegebenenfalls von Vorteil, die Reaktion in Gegenwart einer Schutzgasatmosphäre wie beispielsweise Argon durchzuführen.
Die Reaktionsdurchführung, Aufarbeitung und Isolierung der Reaktionsprodukte erfolgt nach allgemein üblichen Methoden.

Alternativ zur Herstellung der erfindungsgemäßen Wirkstoffe mit Hilfe des erfindungsgemäßen Herstellungsverfahrens sind verschiedene weitere Herstellungsverfahren zur Herstellung der erfindungsgemäßen Wirkstoffe denkbar.

So erhält man erfindungsgemäße Wirkstoffe der Formel (I) beispielsweise auch, wenn man α-Cyanozimtsäureester in Gegenwart von Basen und in Gegenwart von Kupfer-(II)-Salzen mit p-Toluol-sulfonylmethylisocyanid umsetzt (vgl. J6-1030-571 oder J6-1200-984) oder wenn man α-substituierte Zimtsäurenitrile in Gegenwart von Natriumhydrid mit Isocyanoessigsäureestern cyclisiert, die so erhältlichen Pyrrol-2-carbonsäureester mit Basen verseift und anschließend thermisch decarboxyliert (vgl. JP 59/212 468) oder wenn man Phenacylaminderivate mit geeignet substituierten Acrylnitril-Derivaten umsetzt (vgl. EP 174 910) oder wenn man 3-Trifluormethyl-4- phenyl-pyrrole mit Ammoniak bei erhöhter Temperatur und erhöhtem Druck umsetzt (vgl. EP 182 738) oder wenn man 3-Cyano-4-phenyl-$\Delta^2$-pyrroline in Gegenwart von Kupfer-II-Salzen oder Eisen-III-salzen oxidiert (vgl. EP 183 217) oder wenn man α-Cyanoacrylsäurederivate mit Isocyanoessigsäureestern in Gegenwart einer Base umsetzt und die so erhältlichen $\Delta^2$-Pyrrolin-2-carbonsäurederivate in einer 2. Stufe in Gegenwart einer Base und in Gegenwart eines Metallsalzkatalysators

oxidativ decarboxyliert (vgl. Deutsche Patentanmeldung P 3 718 375 vom 02.06.1987).

Die erfindungsgemäßen Wirkstoffe weisen eine starke wirkung gegen Schädlinge auf und können zur Bekämpfung von unerwünschten Schadorganismen praktisch eingesetzt werden. Die Wirkstoffe sind für den Gebrauch als Pflanzenschutzmittel insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Krankheiten im Obst- und Gemüseanbau, wie beispielsweise gegen den Erreger des Bohnengrauschimmels (Botrytis cinerea), oder zur Bekämpfung von Reiskrankheiten, wie beispielsweise gegen den Erreger der Reisfleckenkrankheit (Pyricularia oryzae) einsetzen.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Gra nulate, Aerosole, Feinstverkapselungen in polymeren Stoffen und in Hüllmassen für Saatgut, sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarb stoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe konnen als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann aus das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0.0001 bis 0,02 % am Wirkungsort erforderlich.

Herstellungsbeispiele

Beispiel 1

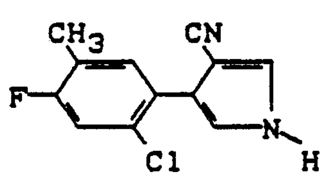

Zu 1,3 g (0.0431 Mol) Natriumhydrid (80 %ig in Mineralöl) in 16 ml Tetrahydrofuran unter einer Argonschutzgasatmosphäre gibt man bei - 10 °C bis - 20 °C tropfenweise unter Rühren eine Lösung von 6,0 g (0.0308 Mol) 3-(4-Fluor-2-chlor-5-methyl-phenyl)-acrylnitril und 7,8 g (0.0431 Mol) p-Toluolsulfonylmethylisocyanid in 20 ml einer Mischung aus Tetrahydrofuran/Dimethylsulfoxid (5:1). Nach beendeter Zugabe läßt man die Reaktionsmischung auf Raumtemperatur kommen, gibt Wasser zu, extrahiert mehrfach mit Essigester, wäscht die vereinigten Essigesterphasen mit Wasser, trocknet über Natriumsulfat und engt im Vakuum ein. Der Rückstand wird durch Chromatographie an Kieselgel (Laufmittel: Cyclohexan/Essigester

5:1) gereinigt.

Man erhält 4,8 g (67 % der Theorie) an 3-Cyano-4-(4-fluor-2-chlor-5-methyl-phenyl)-pyrrol vom Schmelzpunkt 132 °C - 133 °C.

In entsprechender Weise und gemäß den allgemeinen Angaben zur Herstellung erhält man die folgenden 3-Cyano-4-phenyl-pyrrol-Derivate der allgemeinen Formel (I):

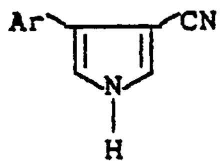 (I)

| Bsp.Nr. | Ar | Schmelzpunkt[°C] |
|---|---|---|
| 2 | 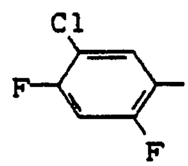 | 184 - 185 |
| 3 | 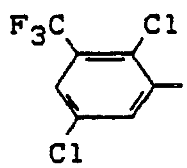 | 155 - 157 |
| 4 | 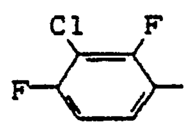 | 218 - 219 |
| 5 | 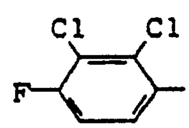 | 188 - 189 |
| 6 | 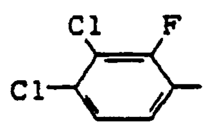 | |

Herstellung der Ausgangsverbindungen

Beispiel II-1

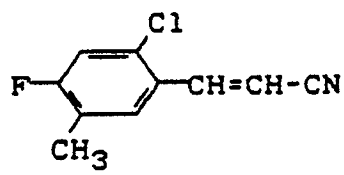

Zu 25,1 g (0.11 Mol) 2-Chlor-3-(2-chlor-4-fluor-5-methyl-phenyl)-propionitril in 100 ml Tetrahydrofuran tropft man bei Raumtemperatur unter Rühren 18,2 g (0,12 Mol) Diazabicycloundecen in 150 ml Tetrahydrofuran, rührt nach beendeter Zugabe 15 Stunden bei Raumtemperatur, filtriert, engt das Filtrat im Vakuum ein, nimmt den Rückstand in Essigester auf, wäscht nacheinander mit 1 normaler Salzsäure und Wasser, trocknet über Natriumsulfat und entfernt das Lösungsmittel in Vakuum.

Man erhält 20,2 g (94 % der Theorie) an 3-(2-Chlor-4-fluor-5-methyl-phenyl)-acrylnitril vom Schmelzpunkt 90 °C - 91 °C.

In entsprechender Weise und gemäß den allgemeinen Angaben zur Herstellung erhält man die folgenden substituierten Zimtsäurenitrile der allgemeinen Formel (II):

$$Ar\text{-}CH{=}CH\text{-}CN \qquad (II)$$

| Bsp.Nr. | Ar | physikalische Eigenschaften |
|---|---|---|
| II-2 | 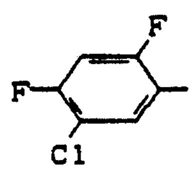 | Kp. 83 - 83 °C/ 0.2 mbar |
| II-3 | 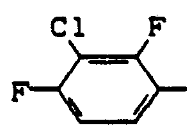 | Fp. 76 - 77 °C |
| II-4 | 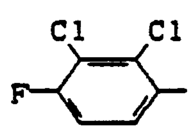 | Fp. 92 - 93 °C |

Beispiel IV-1

a) In einer Rührapparatur werden 537 g 2,4-Dichlortrifluormethoxy-benzol bei 20 °C vorgelegt und 604 g Nitriersäure (33 Gew.-% Salpetersäure, 67 Gew.-% Schwefelsäure) zugetropft. Anschließend wird 1 Stunde bei 40 °C nachgerührt. Nach Austragen der Nitriermischung aus Eis wird mit Dichlormethan

extrahiert, die organische Phase getrocknet und einer Feindestillation unterworfen. Im Siedebereich 131 bis 134° C bei 18 mbar gehen 551 g 3-Nitro-4,6-dichlor-trifluormethoxy-benzol mit einem Brechungsindex $n_D^{20}$ von 1,5291 über.

b) In einer Rührapparatur werden 150 g Kaliumfluorid in 250 ml Tetramethylensulfon vorgelegt und suspendiert. Anschließend wird bei einem Vakuum von 18 mbar 30 ml Lösungsmittel abdestilliert, um die Apparatur und die Einsatzmaterialien wasserfrei zu bekommen. Dann werden 110 g 3-Nitro-4,6-dichlortrifluormethoxy-benzol unter Feuchtigkeitsausschluß zugesetzt und für 6 Stunden bei 190° C Innentemperatur gerührt. Die auf ca. 100° C abgekühlte Reaktionslösung wird dann grob destilliert, wobei das Produkt bis zu einem Siedepunkt von 140° C bei 20 mbar gesammelt wird. Das erhaltene Destillat wird zweimal mit je 100 ml kaltem Wasser gewaschen, anschließend getrocknet und einer Feindestillation unterworfen. Bei einem Siedepunkt von 66 bis 68° C bei 10 mbar werden 27 g 3-Nitro-4,6-difluor-trifluormethoxy-benzol erhalten.

c) In einer Hydrierapparatur werden 27 g 3-Nitro-4,6-difluor-trifluormethoxy-benzol in 150 ml Tetrahydrofuran in Gegenwart von 3 g Raney-Nickel bei 25 bis 45° C mit 30 bis 50 bar Wasserstoff hydriert. Nach Beendigung der Wasserstoffaufnahme wird das Gemisch filtriert und das Filtrat destilliert. Es werden 19 g 3-Amino-4,6-difluor-trifluormethoxy-benzol mit einem Siedepunkt von 82 bis 83° C bei 20 mbar erhalten.

Beispiel IV-2

a) Zu 162 g 2,6-Difluor-benzotrifluorid werden bei 50 bis 55° C 220 g Nitriersäure (33 Gew.-% Salpetersäure, 67 Gew.-% Schwefelsäure) getropft. Nach einer Stunde Nachreaktionszeit wird auf Eis gegossen, die organische Phase mit Dichlormethan abgetrennt und nach Trocknung destilliert. Es werden 178 g 3-Nitro-2,6-difluor-benzotrifluorid mit einem Siedepunkt von 96 bis 100° C bei 22 mbar und einem Brechungsindex $n_D^{20}$ von 1,4570 erhalten.

b) In einer Hydrierapparatur werden 178 g der gemäß a) erhaltenen Nitroverbindung in 680 ml Tetrahydrofuran zusammen mit 15 g Raney-Nickel vorgelegt und bei 25 bis 45° C mit 30 bis 50 bar Wasserstoffdruck hydriert. Wenn kein Wasserstoff mehr aufgenommen wird, wird abgekühlt und entspannt. Durch Destillation der filtrierten Reaktionsmischung werden 147 g 2,6-Difluor-3-amino-benzotrifluorid mit einem Siedepunkt von 89 bis 90° C bei 18 mbar erhalten.

Beispiel IV-3

In einem Edelstahlautoklaven werden 200 ml Tetrahydrofuran und 50 g 2,4,6-Trifluorbenzotrifluorid vorgelegt und 30 ml flüssiger Ammoniak aufgedrückt. Anschließend wird unter Rühren für 6 Stunden auf 120° C erhitzt. Nach Abkühlen und Entspannen wird die Reaktionsmischung einer fraktionierten Destillation unterworfen. Bei einem Siedepunkt von 57 bis 58° C bei 12 mbar werden 15 g 2-Amino-4,6-difluor-benzotrifluorid erhalten. Nach einem kleinen Zwischenlauf gehen bei einem Siedepunkt von 103 bis 105° C bei 16 mbar 32 g 2,6-Difluor-4-amino-benzotrifluorid über. Der Schmelzpunkt des erhaltenen 2,6-Difluor-4-amino-benzotrifluorids liegt bei 66° C.

Beispiel IV-4

In einem Edelstahlautoklaven werden 500 g 3,4,5-Trifluor-benzotrifluorid vorgelegt, 2000 ml Tetrahydrofuran zugegeben und 150 g flüssiger Ammoniak aufgedrückt. Unter Rühren wird der Autoklave für 5 Stunden auf 120 bis 130° C erhitzt, dann abgekühlt und bei 20° C entspannt. Durch Destillation werden neben dem Lösungsmittel und Ausgangsmaterial 272 g 3,5-Difluor-4-amino-benzotrifluorid mit einem Siedepunkt von 58 bis 60° C bei 16 mbar erhalten.

Beispiel IV-5

In einem Autoklaven werden 200 g 2,3,4-Trifluorbenzotrifluorid in 500 ml Tetrahydrofuran vorgelegt und 60 ml flüssiger Ammoniak aufgedrückt. Nach 6 Stunden Rühren bei 130° C (maximaler Druck 18 bar) wird abgekühlt und entspannt. Durch Destillation werden bei einem Siedepunkt von 60 bis 64° C bei 26 mbar 72

g 2-Amino-3,4-difluor-benzotrifluorid und bei einem Siedepunkt von 92 bis 93° C bei 26 mbar 92 g 2,3-Difluor-4-amino-benzotrifluorid erhalten.

Beispiel V-1

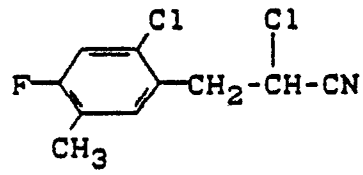

Zu 19,6 g (0,12 Mol) 2-Chlor-4-fluor-5-methylanilin in 40 ml Aceton gibt man 40 ml 25prozentige Salzsäure und 28,8 ml (0,37 Mol) Acrylsäurenitril, tropft dann bei 0 °C bis 10 °C unter Rühren innerhalb einer Stunde 8,7 g (0,13 Mol) Natriumnitrit in 17 ml Wasser zu, rührt eine weitere Stunde bei 0 °C bis 10 °C und gibt dann mehrere Portionen Kupfer-II-oxid-Pulver zu, wobei eine heftige Stickstoffgasentwicklung zu beobachten ist. Nach beendeter Gasentwicklung rührt man weitere 15 Stunden bei Raumtemperatur, gibt dan Dichlormethan zu, wäscht mit Wasser, trocknet über Natriumsulfat, engt im Vakuum ein und reinigt den öligen Rückstand durch Chromatographie an Kieselgel (Laufmittel: Cyclohexan/Essigester 1:1).

Man erhält 25,3 g (90 % der Theorie) an 2-Chlor-3-(2-chlor-4-fluor-5-methylphenyl)-propionitril als Öl.

$^1$H-NMR ($CDCl_3$/TMS) :
$\delta$ = 2,3 (3 H); 3,4 (2 H);
4,7 (1 H); 7,0-7,2 (2 H) ppm.

In entsprechender Weise und gemäß den allgemeinen Angaben zur Herstellung erhält man die folgenden $\alpha$-Chlor-$\beta$-phenyl-propionitrile der Formel (V):

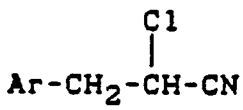 (V)

| Bsp.Nr. | Ar | physikalische Eigenschaften |
|---|---|---|
| V-2 | 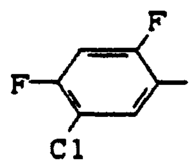 | $^1H$-NMR*): 3,3; 4,6; 7,3; 7,4 |
| V-3 | 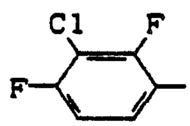 | Kp. 75-77 °C/ 0,15 mbar |
| V-4 | 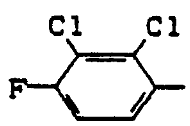 | Kp. 86-88 °C/ 0,1 mbar |

*) Die $^1H$-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

## Anwendungsbeispiele

In dem folgenden Anwendungsbeispiel wurde die nachstehend aufgeführte Verbindung als Vergleichsubstanz eingesetzt:

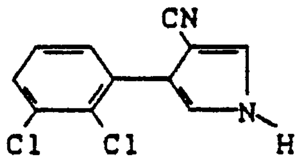

(A)

3-Cyano-4-(2,3-dichlorphenyl)-pyrrol

(vgl. EP 174 910 sowie EP 236 272).

Beispiel A

Botrytis-Test (Bohne)/protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteile Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20 °C aufgestellt. 3 Tage nach der Inokulation wird die Größe der Befallsflecken auf den Blättern ausgewertet.

Eine deutliche Überlegenheit in der Wirksamkeit gegenüber dem Stand der Technik zeigen in diesem Test z.B. die Verbindungen gemäß Herstellungsbeispielen 4 + 5.

Beispiel B

Leptosphaeria nodorum-Test (Weizen) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Sporensuspension von Leptosphaeria nodorum besprüht. Die Pflanzen verbleiben 48 Stunden bei 20° C und 100 % relativer Luftfeuchtigkeit in einer Inkubationskabine.

Die Pflanzen werden in einem Gewächshaus bei einer Temperatur von ca. 15° C und einer relativen Luftfeuchtigkeit von ca. 80 % aufgestellt.

10 Tage nach der Inokulation erfolgt die Auswertung.

Eine deutliche Überlegenheit in der Wirksamkeit gegenüber dem Stand der Technik zeigen bei diesem Test z.B. die Verbindungen gemäß den Herstellungsbeispielen: 4, 5 und 6.

**Ansprüche**

1. 3-Cyano-4-phenyl-pyrrol-Derivate der allgemeinen Formel (I),

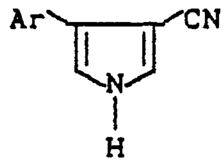

(I)

in welcher
Ar für drei- bis fünffach substituiertes Phenyl steht.

2. 3-Cyano-4-phenyl-pyrrol-Derivate gemäß Anspruch 1, wobei in der Formel (I)
Ar für drei- bis fünffach, gleich oder verschieden substituiertes Phenyl steht, wobei als Substituenten genannt seien: Halogen, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen, jeweils zweifach verknüpftes Dioxyalkylen oder Halogendioxyalkylen mit jeweils 1 oder 2 Kohlenstoffatomen und gegebenenfalls 1 bis 4 gleichen oder verschiedenen Halogenatomen.

3. 3-Cyano-4-phenyl-pyrrol-Derivate gemäß Anspruch 1, wobei in der Formel (I)
Ar für drei- bis fünffach, gleich oder verschieden substituiertes Phenyl steht, wobei als Substituenten besonders bevorzugt sind: Fluor, Chlor, Brom, Methyl, Methoxy, Methylthio, Trifluormethyl, Trifluormethoxy, Trifluormethylthio, jeweils zweifach verknüpftes Dioxymethylen oder Difluordioxymethylen.

4. 3-Cyano-4-phenyl-pyrrol-Derivate gemäß Anspruch 1, wobei in der Formel (I)
Ar für drei- bis fünffach, gleich oder verschieden substituiertes Phenyl steht, wobei mindestens ein Substituent Fluor ist und wobei als weitere Substituenten infrage kommen: Chlor, Brom, Methyl, Methoxy, Methylthio, Trifluormethyl, Trifluormethoxy, Trifluormethylthio oder Difluordioxymethylen.

5. Verfahren zur Herstellung von 3-Cyano-4-phenyl-pyrrol-Derivaten der Formel (I),

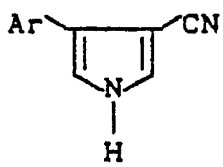

(I)

in welcher
Ar für drei- bis fünffach substituiertes Phenyl steht,
dadurch gekennzeichnet, daß man substituierte Zimtsäurenitrile der Formel (II),
Ar-CH=CH-CN (II)
in welcher
Ar die oben angegebene Bedeutung hat,
mit Sulfonylmethylisocyaniden der Formel (III),
$R-SO_2-CH_2-NC$ (III)
in welcher
R für Alkyl oder für gegebenenfalls substituiertes Aryl steht,
in Gegenwart einer Base und gegebenenfalls in Gegenwart eines Verdünnungsmittels umsetzt.

6. Schädlingsbekämpfungsmittel, gekennzeichnet durch einen Gehalt an mindestens einem 3-Cyano-4-phenyl-pyrrol-Derivat der Formel (I) nach den Ansprüchen 1 und 5.

7. Verwendung von 3-Cyano-4-phenyl-pyrrol-Derivaten der Formel (I) nach den Ansprüchen 1 und 5 zur Bekämpfung von Schädlingen.

8. Verfahren zur Bekämpfung von Schädlingen, dadurch gekennzeichnet, daß man 3-Cyano-4-phenyl-pyrrol-Derivate der Formel (I) nach den Ansprüchen 1 und 5 auf Schädlinge und/oder ihren Lebensraum einwirken läßt.

9. Verfahren zur Herstellung von Schädlingsbekämpfungsmitteln, dadurch gekennzeichnet, daß man 3-Cyano-4-phenyl-pyrrol-Derivate der Formel (I) nach den Ansprüchen 1 und 5 mit Streckmitteln und/oder oberflächenaktiven Mitteln vermischt.

10. Substituierte Zimtsäurenitrile der Formel (II),

Ar-CH = CH-CN (II)

in welcher

Ar für drei- bis fünffach substituiertes Phenyl steht.

11. Verfahren zur Herstellung von substituierten Zimtsäurenitrilen der Formel (II),

Ar-CH = CH-CN (II)

in welcher

Ar für drei- bis fünffach substituiertes Phenyl steht,

dadurch gekennzeichnet, daß man

a) substituierte Aniline der Formel (IV),

$Ar-NH_2$ (IV)

in welcher

Ar die oben angegebene Bedeutung hat,

zunächst in einer ersten Stufe mit Acrylnitril unter üblichen Diazotierungsbedingungen, in Gegenwart eines geeigneten Metallsalz-Katalysators und gegebenenfalls in Gegenwart eines geeigneten Verdünnungsmittels bei Temperaturen zwischen -20° C und 50° C umsetzt und dann in einer 2. Stufe die so erhältlichen substituierten α-Chlor-β-phenyl-propionitrile der Formel (V),

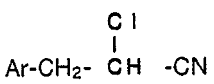 (V)

in welcher

Ar die oben angegebene Bedeutung hat,

mit Basen, gegebenenfalls in Gegenwart eines Verdünnungsmittels bei Temperaturen zwischen 0° C und 50° C dehydrohalogeniert oder alternativ

b) substituierte Benzaldehyde der Formel (VI),

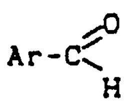 (VI)

in welcher

Ar die oben angegebene Bedeutung hat,

mit Cyanessigsäure der Formel (VII),

$NC-CH_2-COOH$ (VII)

in Gegenwart einer Base und gegebenenfalls in Gegenwart eines geeigneten Verdünnungsmittels bei Temperaturen zwischen 50° C und 120° C kondensiert und gleichzeitig decarboxyliert.